# FASCICULE DE BREVET EUROPEEN

(11) **EP 3 539 601 B1**
(45) Date de publication et mention de la délivrance du brevet: **12.05.2021**
(21) Numéro de dépôt: 19160034.5
(22) Date de dépôt: 28.02.2019
(51) Int. Cl.: A61M 16/00, A61M 16/16

(54) **ENSEMBLE DE VENTILATION DE PATIENT COMPRENANT UNE STATION D'ACCUEIL ET UN VENTILATEUR MÉDICAL**
PATIENTEN-BEATMUNGSEINHEIT, DIE EINE AUFNAHMESTATION UND EIN MEDIZINISCHES BEATMUNGSGERÄT UMFASST
PATIENT VENTILATION ASSEMBLY COMPRISING A DOCKING STATION AND A MEDICAL VENTILATOR

(30) Priorité: 14.03.2018 FR 1852183
(43) Date de publication de la demande: 18.09.2019
(73) Titulaire: Air Liquide Medical Systems, 92160 Antony (FR)
(72) Inventeur: DAVOINE, Romain, 92182 Antony (FR)
(74) Mandataire: Air Liquide

(56) Documents cités:
- EP-A1- 2 536 313
- EP-A1- 2 671 607
- FR-A1- 2 901 998
- US-A1- 2006 055 069

## Description

La présente invention porte sur un ensemble de ventilation de patient comprenant une station d'accueil avec des moyens d'accrochage et un ventilateur médical avec des moyens d'arrimage qui coopèrent avec les moyens d'accrochage de la station d'accueil de sorte de solidariser le ventilateur à la station d'accueil.

Les appareils de ventilation, encore appelés appareils d'assistance respiratoire ou plus simplement ventilateurs médicaux, sont couramment utilisés pour fournir un gaz respiratoire, tel de l'air ou de l'air enrichi en oxygène, aux patients souffrant de troubles ou déficiences respiratoires.

Une installation de ventilation comprend généralement une station d'accueil avec une embase ou socle qui est surmontée d'un ventilateur médical incorporant une micro-soufflante, encore appelée turbine ou compresseur, servant à générer un flux de gaz respiratoire, et éventuellement d'un humidificateur de gaz qui sert à humidifier le gaz fourni par le ventilateur.

Ainsi, le document FR-A-3008621 enseigne un tel ensemble de ventilation de patient. Sont prévus également des moyens d'interconnexion électrique et mécanique pour solidariser mécaniquement le ventilateur et l'humidificateur à l'embase de la station d'accueil et pour assurer une alimentation en courant électrique de ceux-ci. Les moyens d'interconnexion mécanique ne sont pas détaillés.

Par ailleurs, FR-A-2901998 enseigne un ensemble de ventilation comprenant un ventilateur et une station d'accueil avec un système de couplage mécanique comprenant des fentes aménagées dans le fond du ventilateur et des picots agencés sur la station d'accueil. Les picots sont fixes et viennent s'engager dans les fentes lors du couplage du ventilateur à la station. L'utilisateur est obligé de faire coulisser l'ensemble du ventilateur pour assurer le couplage mécanique, ce qui est fastidieux et peu pratique, compte tenu du poids du ventilateur.

En outre, EP-A-2671607 propose un respirateur d'urgence conçu pour être fixé à une embase murale fixée à une paroi verticale. La solidarisation se fait via un cliquet pivotant et manœuvrable via une poignée. Ce type de système à fixation par pivotement ne convient pas à un ensemble de ventilation comprenant un ventilateur et une station d'accueil destinée à reposer sur une surface horizontale, de type table ou analogue, et surmontée du ventilateur, c'est-à-dire lorsque la station d'accueil forme une embase ou socle surmontée par le ventilateur, car un couplage par pivotement n'est pas aisé dans ce cas pour l'utilisateur.

Le problème qui se pose est de proposer une installation de ventilation avec un système d'interconnexion mécanique qui permette à un utilisateur, tel un patient, de coupler et de découpler aisément et rapidement le ventilateur médical et la station d'accueil, et qui permette en outre un bon maintien en position du ventilateur sur la station d'accueil, la station d'accueil formant alors une embase ou socle surmontée par le ventilateur, lorsqu'il y est raccordé mécaniquement de manière à assurer un bon fonctionnement de l'appareil en évitant les déconnexions électriques intempestives.

La solution de l'invention porte sur un ensemble de ventilation de patient comprenant une station d'accueil et un ventilateur médical, dans lequel :
- la station d'accueil comprend des moyens d'accrochage,
- le ventilateur comprend des moyens d'arrimage coopérant avec les moyens d'accrochage de la station d'accueil, lorsque le ventilateur est positionné sur ladite station d'accueil, c'est-à-dire lorsque la station d'accueil forme une embase ou socle surmontée par le ventilateur, de manière à solidariser le ventilateur à la station d'accueil, dans lequel :
- les moyens d'arrimage du ventilateur comprennent au moins une ouverture aménagée dans une paroi de fond dudit ventilateur, et
- les moyens d'accrochage de la station d'accueil comprennent au moins un élément à crochet, ledit au moins un élément à crochet venant s'insérer dans ladite au moins une ouverture du ventilateur et prendre appui sur au moins une surface interne du ventilateur pour maintenir ledit ventilateur solidaire de la station d'accueil.

Selon l'invention, le ou les éléments à crochet sont mobiles en translation entre au moins :
i) une position de couplage dans laquelle le ou les éléments à crochet prennent appui sur au moins une surface interne du ventilateur pour maintenir ledit ventilateur solidaire de la station d'accueil, et
ii) une position de découplage dans laquelle le ou les éléments à crochet ne sont pas en appui sur une surface interne du ventilateur de sorte de pouvoir désolidariser le ventilateur de la station d'accueil, et
les moyens d'accrochage de la station d'accueil comprennent en outre des moyens de rappel élastique (i.e. retour élastique) coopérant avec le ou les éléments à crochet de manière à le ou les rappeler élastiquement en position de couplage.

Selon le cas, l'ensemble de ventilation de l'invention peut comprendre l'une ou plusieurs des caractéristiques techniques suivantes :
- les moyens d'arrimage du ventilateur comprennent une pluralité d'ouvertures aménagées dans la paroi de fond dudit ventilateur, et les moyens d'accrochage de la station d'accueil comprennent une pluralité d'éléments à crochet.
- le nombre d'ouvertures aménagées dans la paroi de fond du ventilateur est compris entre 2 et 10, de préférence de 3 à 6, typiquement de l'ordre de 4.
- le nombre d'éléments à crochet de la station d'accueil est compris entre 2 et 10, de préférence de 3 à 6, typiquement de l'ordre de 4.
- le nombre d'ouvertures aménagées dans la paroi de fond dudit ventilateur est égal au nombre d'éléments à crochet de la station d'accueil.
- la ou les ouvertures aménagées dans la paroi de fond du ventilateur sont oblongues.
- la ou les ouvertures aménagées dans la paroi de fond du ventilateur sont des fentes.
- dans la position de couplage, le ou les éléments à crochet, en particulier via leur extrémité en forme de crochet, prennent appui sur la surface interne de la paroi de fond du ventilateur pour maintenir le ventilateur solidaire de la station d'accueil.
- les éléments à crochet comprennent chacun une tête avec une surface externe pentue.
- les éléments à crochet comprennent chacun un bras-porteur se projetant vers le haut et surmonté de la tête à surface externe pentue.
- la pente de la tête de chaque élément à crochet forme un angle compris entre 15° et 45° par rapport à l'axe du bras-porteur.
- les éléments à crochet comprennent chacun une extrémité formant crochet pour permettre un arrimage sur une paroi du ventilateur, de préférence la paroi de fond.
- dans la position de couplage, la tête des éléments à crochet fait saillie vers l'intérieur du ventilateur au travers des ouvertures pratiquées dans la paroi de fond du ventilateur.
- le ventilateur comprend une carcasse externe rigide comprenant la paroi de fond surmontée par une ou plusieurs parois latérales ou périphériques.
- le ventilateur comprend une carcasse externe rigide en matériau polymère, i.e. en plastique.
- la tête à surface externe pentue ou arrondie dudit au moins un élément à crochet vient coopérer avec un rebord de ladite au moins une ouverture aménagée dans la paroi de fond du ventilateur, lorsque le ventilateur est positionné par un utilisateur sur la station d'accueil, de manière à repousser mécaniquement ledit au moins élément à crochet en direction de la position de découplage, ce qui permet de fixer/coupler le ventilateur à la station d'accueil.
- les moyens de rappel élastique comprennent au moins un ressort.
- la station d'accueil est conçue pour être positionnée horizontalement sur un support, tel un meuble, par exemple une table, un plateau de chariot mobile ou autre.
- la station d'accueil est conçue pour former une embase ou socle surmontée par le ventilateur.
- les éléments à crochet sont mobiles en translation horizontale.
- la station d'accueil comprend en outre des moyens de libération, actionnables manuellement, coopérant avec les éléments à crochet de manière à les déplacer vers la position de découplage, lorsqu'ils sont actionnés manuellement par un utilisateur.
- les moyens de libération comprennent un bouton-poussoir.
- le bouton-poussoir est actionnable par un utilisateur pour libérer le ventilateur, c'est-à-dire le bouton-poussoir agit, directement ou indirectement, sur les éléments à crochet pour les déplacer vers leur position de découplage.
- les moyens de libération comprennent un bouton-poussoir mobile en translation.
- les moyens de libération comprennent un bouton-poussoir mobile en translation horizontale, par exemple un bouton-tiroir à poignée à tirer pour libérer.
- le ventilateur médical et la station d'accueil sont configurés, i.e. conçus et adaptés, pour permettre que le couplage du ventilateur médical à la station d'accueil se fasse selon une direction de couplage sensiblement verticale.
- la station d'accueil comprend une embase avec au moins un premier emplacement configuré pour recevoir le ventilateur.
- la station d'accueil comprend une embase avec au moins un second emplacement configuré pour recevoir un humidificateur de gaz.
- le ventilateur comprend une micro-soufflante pour fournir un gaz respiratoire, en particulier de l'air ou de l'air enrichi en oxygène.
- la station d'accueil comprend en outre des moyens d'interconnexion électrique aptes à et conçu pour assurer une alimentation électrique du ventilateur lorsqu'il est couplé à la station d'accueil, en particulier lorsqu'il est positionné dans le premier emplacement.
- les premiers moyens d'interconnexion mécanique permettent d'opérer une solidarisation du ventilateur à l'embase lorsqu'il est positionné dans le premier emplacement.
- la station d'accueil comprend des moyens à carte électronique comprenant au moins une carte électronique aptes à et conçus pour permettre une communication d'informations entre ladite carte électronique et le ventilateur lorsqu'il est couplé à la station d'accueil,
- la station d'accueil comprend des moyens d'alimentation électrique.
- la station d'accueil comprend au moins une batterie, notamment rechargeable.
- les moyens d'alimentation électrique sont connectés électriquement aux moyens à carte électronique et aux moyens d'interconnexion électrique.
- les moyens à carte électronique sont agencés dans l'embase de la station d'accueil.
- la carte électronique permet d'assurer une communication d'informations du type état des batteries ou état de charge (branché, en défaut, en charge, en décharge...), état des accessoires, état du ventilateur (vitesse de la turbine, accessoires branché...) et/ou état de l'humidificateur (en cours de chauffe, à quelle puissance...).
- les moyens d'interconnexion électrique comprennent au moins un fil électrique, c'est-à-dire un câble électrique ou analogue.
- les moyens d'interconnexion électrique comprennent des connecteurs électriques, en particulier au moins un connecteur mâle et au moins un connecteur femelle coopérant l'un avec l'autre.
- la carte électronique assure la partie logique en effectuant un traitement des informations reçues et générant des informations à émettre qui transitent par les moyens d'interconnexion électrique, c'est-à-dire fil, câble ou analogue.
- les moyens d'interconnexion électrique comprennent au moins un bus numérique permettant l'échange d'informations et le pilotage de la station d'accueil par le ventilateur.
- les moyens d'échange de la carte électronique sont aptes à et conçus pour assurer un échange d'informations sur l'état de la station d'accueil et/ou de la ou des batteries, en particulier des informations de type alimentation ou état de la batterie.
- la carte électronique permet d'assurer un pilotage et une gestion de la ou des batteries, en particulier de surveillance de l'état de la ou des batteries.
- la carte électronique comprend au moins un microprocesseur, en particulier un microcontrôleur.
- le second emplacement configuré pour recevoir un humidificateur de gaz comprend des seconds moyens de positionnement mécanique
- les seconds moyens de positionnement mécanique sont conçus pour permettre de positionner de manière réversible l'humidificateur au sein du second emplacement de l'embase, c'est-à-dire que l'humidificateur peut y être fixé ou retiré au gré des besoins de l'utilisateur, en même temps que le ventilateur (i.e. pas d'extraction de l'humidificateur sans extraction du ventilateur), en particulier on retire l'humidificateur lorsque le gaz délivré au patient n'a pas besoin d'être humidifié.
- les moyens d'alimentation électrique comprennent une ou plusieurs batteries d'alimentation électriques, typiquement de 1 à 4 batteries, ou plus si nécessaire.
- les batteries d'alimentation électrique sont choisies dans le groupe des batteries électriques, des accumulateurs électriques et des piles électriques, par exemple des batteries regroupant des accumulateurs de base lithium ou nickel.
- la ou les batteries est/sont rechargeables électriquement.
- la station d'accueil comprend en outre des moyens de fourniture de courant reliés électriquement auxdits moyens d'interconnexion électrique de manière à alimenter lesdits moyens d'interconnexion électrique en courant électrique issu d'une source de courant extérieure.
- les moyens de fourniture de courant permettent d'alimenter le ventilateur, lorsqu'il est positionné dans ledit premier emplacement, en courant électrique venant d'une source électrique extérieure, tel le secteur, et/ou d'opérer une charge électrique de la ou des batteries.
- les moyens de fourniture de courant comprennent des moyens de raccordement au courant secteur comprenant une prise électrique et un ou plusieurs fils électriques.
- les moyens de connexion électrique comprennent des connecteurs électriques ou analogues par exemple des connecteurs plats ou à lames.
- les moyens de connexion électrique sont alimentés par au moins une batterie électrique ou par les moyens de fourniture de courant alimentant la station en courant issu d'une source de courant extérieure.
- la source de courant extérieure est le réseau-secteur qui délivre un courant de tension comprise entre 100 et 230 V.
- il comprend en outre une interface homme/machine comprenant un ou des organes de commande, en particulier des boutons ou touches, et/ou un ou des dispositifs visuels ou optiques, en particulier des voyants lumineux ou un afficheur numérique.
- il comprend en outre un humidificateur de gaz en communication fluidique avec le ventilateur de sorte d'humidifier le gaz délivré par le ventilateur.
- il comprend en outre un humidificateur de gaz agencé sur la station d'accueil.

La présente invention va maintenant être décrite plus en détail en références aux Figures annexées parmi lesquelles :
- la Figure 1 est un schéma d'un mode de réalisation d'un ensemble de ventilation à station d'accueil et ventilateur médical selon la présente invention,
- la Figure 2 est un schéma d'un mode de réalisation d'un ensemble de ventilation à station d'accueil, ventilateur médical et humidificateur selon la présente invention,
- la Figure 3 est un schéma d'un mode de réalisation de la station d'accueil de l'ensemble de l'invention des Figures 1 et 2,
- la Figure 4 représente un mode de réalisation d'une IHM agencée dans la station d'accueil d'un ensemble de ventilation selon la présente invention,
- la Figure 5 est une vue en coupe de la station d'accueil d'un ensemble de ventilation selon la présente invention,
- les Figures 6A-6C illustrent une mise en position du ventilateur sur la station d'accueil d'un ensemble de ventilation selon la présente invention, avec interconnexion électrique,
- la Figure 7 est une vue agrandie d'un mode de réalisation d'un élément à crochet servant à la fixation du ventilateur sur la station d'accueil d'un ensemble de ventilation selon la présente invention,
- les Figures 8A-8C illustrent une mise en position du ventilateur sur la station d'accueil d'un ensemble de ventilation selon la présente invention, avec interconnexion mécanique entre les éléments à crochet et les ouvertures du fond du ventilateur, et
- la Figure 9 représente un mode de réalisation d'un bouton-poussoir d'une station d'accueil d'un ensemble de ventilation selon l'invention, en particulier celui des Figures 1 et 2.

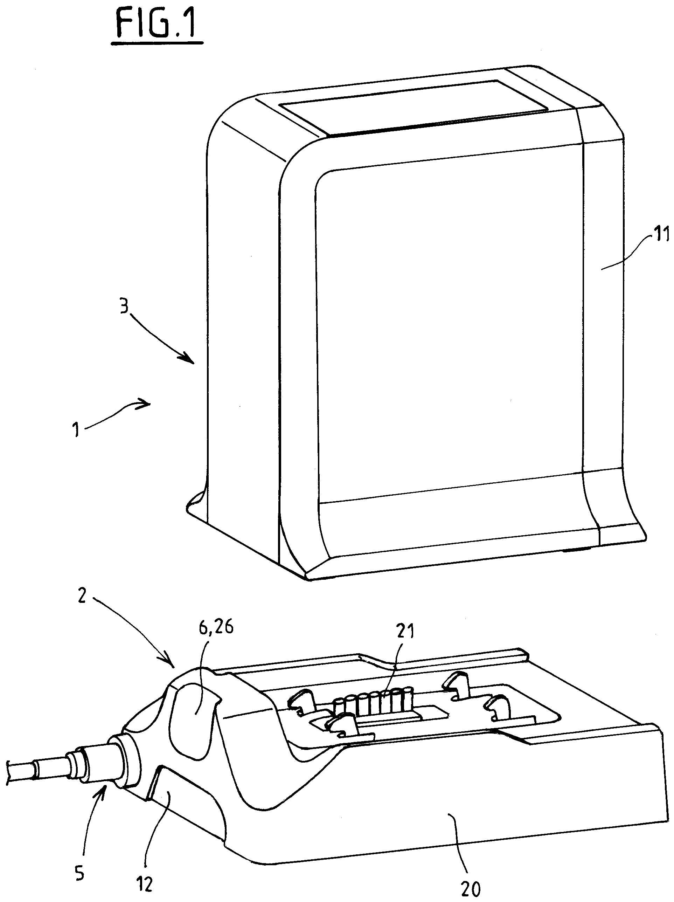

La Figure 1 représente un premier mode de réalisation d'un ensemble de ventilation 1 selon l'invention comprenant une station d'accueil 2 et ventilateur médical 3 comprenant une carcasse rigide 11, par exemple en plastique, renfermant une micro-soufflante pour fournir un gaz respiratoire, tel de l'air, destiné à un patient qui est raccordé à cet ensemble de ventilation 1, via un conduit flexible et une interface respiratoire (non représentés), tel un masque respiratoire ou analogue.

La station d'accueil 2 comprenant une embase 20 intégrant une carte électronique 24 à microprocesseur et une ou plusieurs batteries d'alimentation électrique (non montrées) comme illustré sur les Figures 1, 2, 3 et 5.

Dans le premier mode de réalisation de la Figure 1, l'ensemble de ventilation 1 ne comprend pas d'humidificateur 4 de gaz.

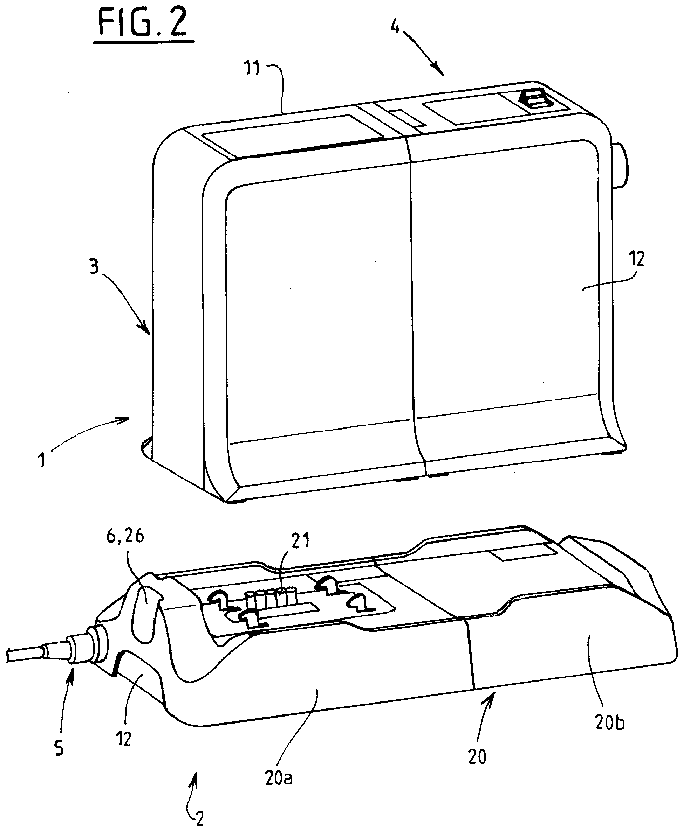

La Figure 2 représente un deuxième mode de réalisation d'un ensemble de ventilation 1 selon la présente invention, identique à celui de la Figure 1, mais comprenant en outre un humidificateur de gaz 4 et une embase 20 modifiée pour recevoir aussi l'humidificateur de gaz 4. Plus précisément, l'embase 20 de la station d'accueil 2 peut être soit agrandie pour recevoir le ventilateur médical 3 et l'humidificateur de gaz 4, soit formée de deux sous-parties 20a, 20b venant se fixer l'une à l'autre, l'une 20a de ces sous-parties 20a, 20b recevant le ventilateur médical 3 et l'autre 20b recevant l'humidificateur de gaz 4.

Une ou plusieurs batteries d'alimentation électrique peuvent être aménagées dans l'une ou l'autre de ces deux sous-parties 20a, 20b.

L'humidificateur de gaz 4 est situé en aval par rapport au ventilateur médical 3, en considérant le trajet du gaz, et en communication fluidique avec celui-ci de manière à pouvoir humidifier le gaz provenant du ventilateur médical 3, avant de l'envoyer vers le patient.

L'humidificateur de gaz 4 comprend lui-aussi une carcasse rigide 12, par exemple en plastique, et renferme une cuve à eau, de préférence amovible, traversée par le flux de gaz, tel de l'air, provenant du ventilateur médical 3. La cuve à eau comprend typiquement une première plaque chauffante en contact avec une seconde plaque chauffante agencée dans le fond de l'humidificateur de gaz 4 et chauffée par une résistance électrique ou analogue de manière à transmettre des calories à l'eau de la cuve pour la chauffer et ainsi générer de la vapeur d'eau servant à humidifier le flux de gaz.

En d'autres termes, l'embase 2 comprend toujours un premier emplacement configuré pour recevoir le ventilateur médical 3 (Fig. 1) et éventuellement un second emplacement configuré pour recevoir l'humidificateur de gaz 4 (Fig. 2).

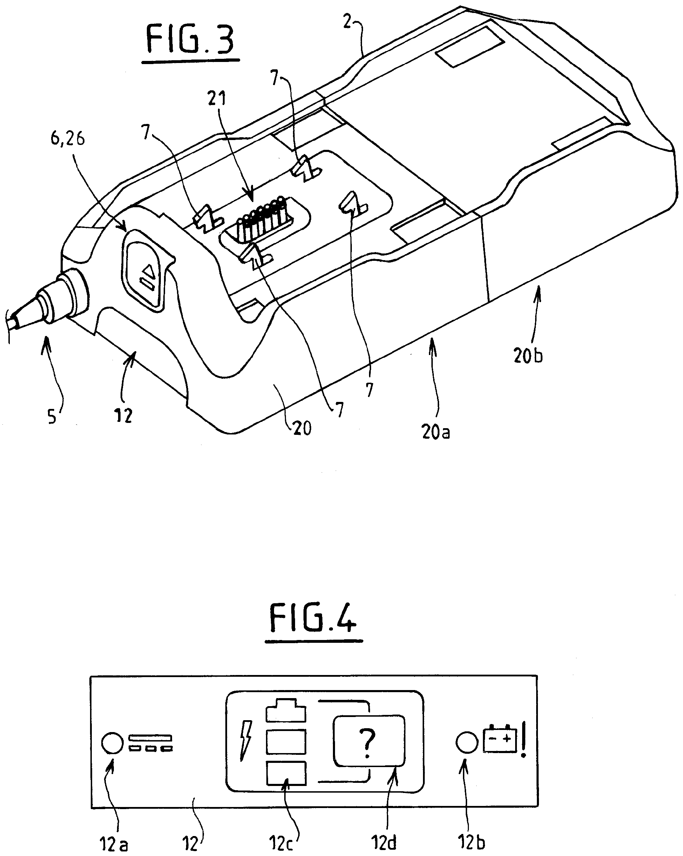

La Figure 3 permet de comprendre que l'embase 20 de la station d'accueil 2 comprenant une enveloppe externe rigide en matière thermoplastique, par exemple ABS ou ABS-PC, et de 1 à 3 mm d'épaisseur. Elle intègre les moyens de connexion électrique et mécanique, comme expliqué ci-après, ainsi qu'une interface homme machine 12 ou IHM (Fig. 4), une carte électronique 24 et une (ou plusieurs) batterie, et des moyens de raccordement électrique externe 5, tel un câble d'alimentation électrique.

D'une façon générale, on prévoit, selon l'invention, des moyens d'interconnexion mécanique aptes à opérer une solidarisation du ventilateur 3 à l'embase 20 au lorsque le ventilateur 3 est mis en position sur la station d'accueil 2. Ces moyens d'interconnexion mécanique sont conçus pour que la solidarisation de du ventilateur 10 à la station d'accueil 2 soit réversible et aisée pour l'utilisateur. De même, la désolidarisation ou découplage du ventilateur 10 de la station d'accueil 2 est aussi aisé et rapide.

Les moyens d'interconnexion mécanique de la présente invention comprennent des moyens d'accrochage 7, 13 agencés sur la station d'accueil 2 coopérant avec des moyens d'arrimage 8, 9, lorsque le ventilateur 3 est positionné sur la station d'accueil 2, c'est-à-dire sur l'embase 20, 20a, de sorte de solidariser le ventilateur 3 à la station d'accueil 2.

Selon la présente invention, les moyens d'arrimage 8, 9 du ventilateur 3 comprennent au une ou plusieurs ouvertures 8 aménagées dans une paroi de fond 9 dudit ventilateur 3, de préférence plusieurs ouvertures 8. Les ouvertures 8 sont préférentiellement des fentes oblongues, notamment de forme rectangulaire, par exemple d'une longueur d'environ 10 à 15 mm, et d'une largeur d'environ 3 à 6 mm.

Par ailleurs, les moyens d'accrochage 7, 13 de la station d'accueil 2 comprennent au moins un élément à crochet 7, de préférence plusieurs éléments à crochet 7.

Les (ou le) éléments à crochet 7 viennent s'insérer dans les (ou la) ouvertures 8, en particulier des fentes, aménagées dans le fond 9 du ventilateur 3, c'est-à-dire la paroi du dessous de la carcasse 11 du ventilateur 3, et prendre appui sur la surface interne 9a du fond 9 du ventilateur 3 pour agripper le ventilateur 3 et le maintenir solidaire de la station d'accueil 2.

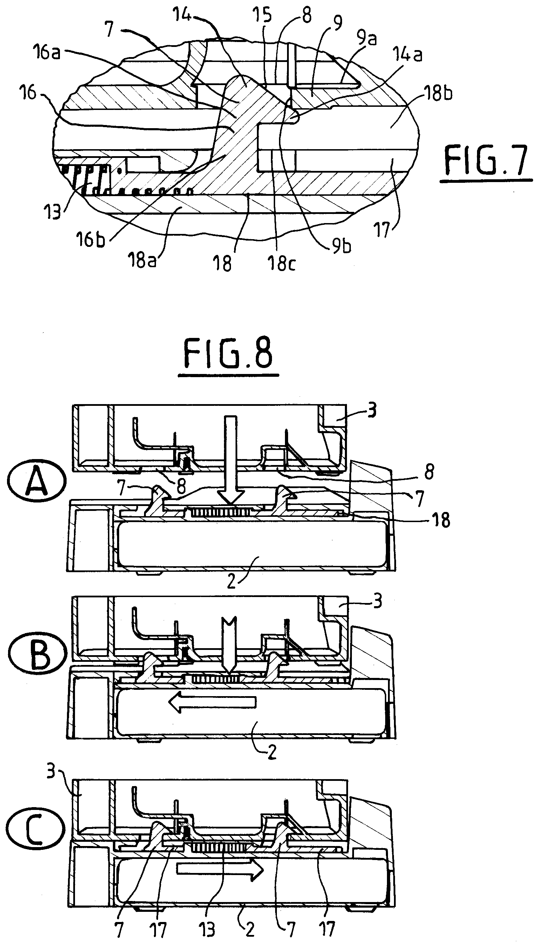

Chaque élément à crochet 7 comporte une tête 14 avec une surface externe pentue 15, de préférence pentue, comme illustré en Figure 7. La tête 14 est située à l'extrémité supérieure 16a d'un bras-porteur 16 se projetant vers le haut à partir d'une structure-socle 17 sur laquelle le bras-porteur 16 est fixé via son extrémité inférieure 16b. Préférentiellement, la surface externe pentue 15 des têtes 14 comprend une pente d'environ 15 à 50°, idéalement de l'ordre de 45°, par rapport à l'axe vertical du bras-porteur 16 supportant la tête 14 à surface externe pentue 15 qui forme un crochet.

La tête 14 de chaque élément à crochet 7 vient coopérer avec un rebord 9b de chaque ouverture 8, telles des fentes, aménagée dans la paroi de fond 9 du ventilateur 3, lorsque le ventilateur 3 est positionné par un utilisateur sur la station d'accueil 2 de manière à repousser mécaniquement l'élément à crochet 7 en direction de la position de découplage.

En effet, lorsque l'utilisateur met en place le ventilateur 3 sur la station d'accueil 2, la tête 14 des éléments à crochet 7 entre en contact mécanique avec le rebord 9b des ouvertures 8, typiquement des fentes, et la force d'appui exercé manuellement par l'utilisateur sur la carcasse 11 du ventilateur 3, typiquement une poussée verticale vers le bas, c'est-à-dire en direction de la station d'accueil 2 comme illustré sur la Figure 8A, se répercute sur la tête 14 des éléments à crochet 7, en particulier sur la surface en pente 15 des têtes 14, ce qui engendre un déplacement translatif des éléments à crochet 7 dans un sens tendant à éloigner les éléments à crochet 7 des rebords 9b.

Pendant ce déplacement en translation et en éloignement, la surface en pente 15 des têtes 14 glisse sur les rebord 9b, comme illustré sur les Fig. 7 et Fig. 8B. Ceci est possible car les éléments à crochet 7 sont mobiles dans l'embase 20 de la station d'accueil 2.

Plus précisément, chaque structure-socle 17 des éléments à crochet 7 est mobile dans un logement de guidage 18, de préférence de forme oblongue, agencé dans l'embase 20 dont les parois qui le délimitent, notamment une paroi de fond 18a, un plafond 18b et deux parois latérales (Fig. 7), peuvent former des rails de guidage pour contrôler les déplacements translatifs de chaque élément à crochet 7, en particulier de sa structure-socle 17, au sein du logement de guidage 18 dans lequel il est aménagé.

Les déplacements translatifs de chaque élément à crochet 7 se font dans un espacement 18c agencé dans le plafond 18b du logement de guidage 18 (Fig. 7).

Une fois que l'extrémité en crochet 14a de chaque tête 14 des éléments à crochet 7 a passé le rebord 9b de l'ouverture 8, le tête 14 est rappelé vers la position de couplage (Fig. 8C) dans laquelle le ou les éléments à crochet 7 prennent appui sur la surface interne 9a, en particulier de la paroi de fond, du ventilateur 3 pour maintenir le ventilateur 3 solidaire de la station d'accueil 2.

En d'autres termes, l'extrémité en crochet 14a de chaque tête 14 vient agripper la surface interne 9a de la paroi de fond 9 du ventilateur 3 de sorte de maintenir le ventilateur 3 solidaire de l'embase 20 de la station d'accueil 2, en position de couplage, comme illustré sur la Figure 8C.

Afin de permettre un rappel élastique des éléments à crochet 7, il est prévu des moyens de rappel élastique 13, tels des ressorts, agencés dans l'embase 20 et coopérant avec les éléments à crochet 7, en particulier avec les structures-socles 17 portant les bras-porteurs 16, de manière à les rappeler élastiquement vers la position de couplage. Avantageusement, la force de rappel exercée par chaque ressort est de l'ordre de 2 à 20 N.

A l'inverse, l'utilisateur doit pouvoir découpler le ventilateur 3 de la station d'accueil 2. Pour ce faire, il est prévu sur la station d'accueil 2, des moyens de libération 6, avantageusement les moyens de libération 6 comprennent un bouton-poussoir 26 servant au déverrouillage du ventilateur 3, comme illustré en Figures 1 à 3 et 9.

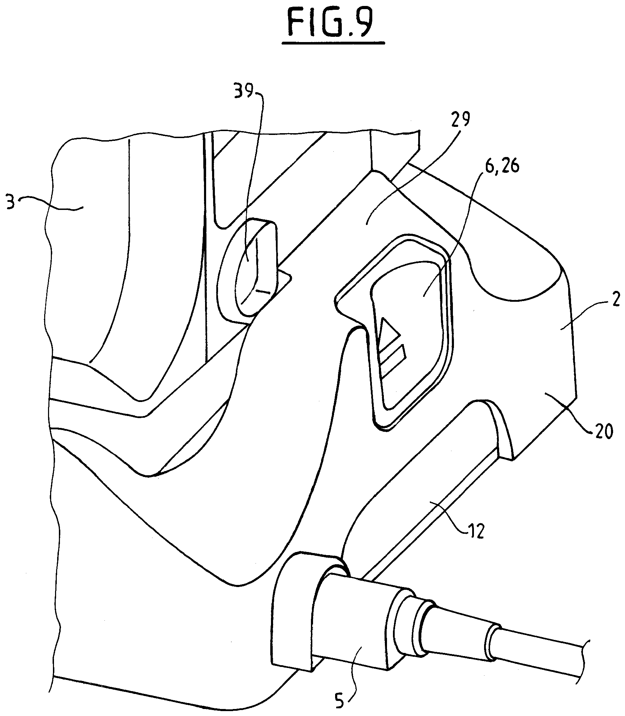

Ce bouton-poussoir 26 (Fig. 9) est actionnable manuellement, lorsque l'utilisateur y exerce une poussée digitale pouvant aller jusqu'à 10 N, et coopère alors avec les éléments à crochet 7 de manière à les déplacer vers une position de découplage, dans laquelle les éléments à crochet 7 sont libérés, c'est-à-dire qu'ils ne sont plus en appui sur la surface interne 9a du fond 9 du ventilateur 3. Ceci permet alors de désolidariser le ventilateur 3 de la station d'accueil 2 puisque l'extrémité en crochet 14a de chaque tête 14 n'agrippe plus alors la surface interne 9a de la paroi de fond 9 du ventilateur 3, ce qui désolidarise alors le ventilateur 3 de la station d'accueil 2. Le ventilateur 3 ainsi libéré peut alors être découplé et retiré de la station d'accueil 2.

Le bouton-poussoir 26 peut avantageusement être un bouton comportant les crochets des structures-socles 17, réalisé en matière thermoplastique, par exemple ABS, POM ou PA, ou encore métallique, ayant une épaisseur comprise entre 1 et 3 mm environ.

Il comprend de 3 à 4 crochets, typiquement 4 crochets.

Par ailleurs, il est prévu aussi des moyens d'interconnexion électrique aptes à opérer une alimentation électrique du ventilateur 3 via l'embase 20 de la station d'accueil 2.

Ces moyens d'interconnexion électrique 21, 22 permettent d'assurer une connexion électrique pour assurer une alimentation électrique du ventilateur 3, tel le courant du réseau secteur, via un câble électrique 5 ou analogue, ou de la ou des batteries, ainsi que le transfert des informations de surveillance de l'alimentation externe et de la ou des batteries contenues dans l'embase 20 de la station d'accueil 2, vers le ventilateur 3.

Les moyens d'interconnexion électrique 21, 22 comprennent des connecteurs électriques, par exemple un connecteur électrique mâle 21 et un connecteur électrique femelle 22 venant s'enficher l'un dans l'autre pour assurer une liaison électrique entre station d'accueil 2 et ventilateur 3, et éventuellement humidificateur 4.

Le connecteur mâle 21 est par exemple agencé sur l'embase 20 de la station d'accueil 2, et le connecteur femelle 22 est par exemple agencé sur le ventilateur 3, comme illustré en Figures 3, 5 et 6A, notamment, ou inversement.

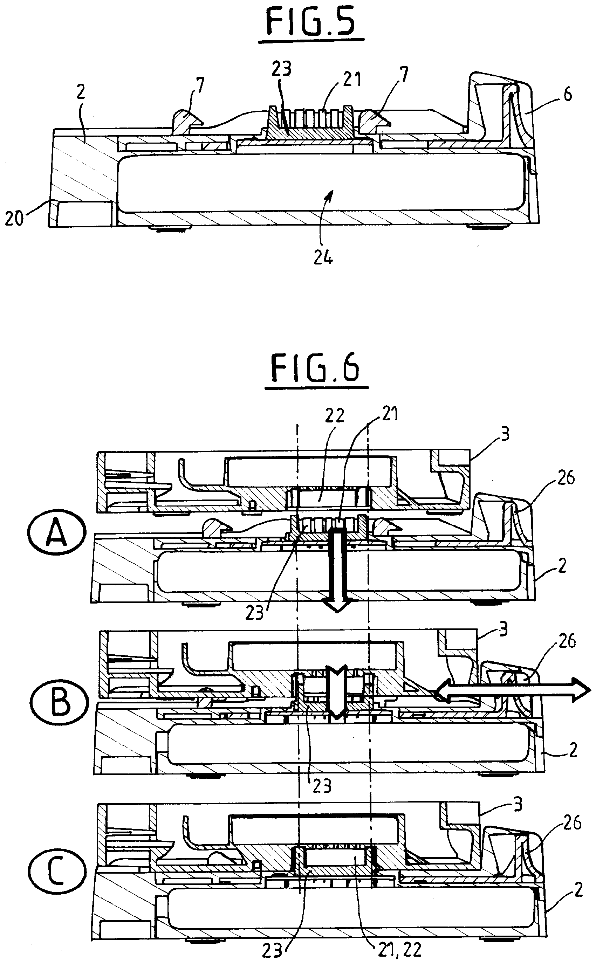

Le ventilateur 3 est donc d'abord pré-positionné sur la station d'accueil 2 par l'utilisateur, par exemple grâce à une forme particulière de l'enveloppe externe rigide la station d'accueil 2, c'est-à-dire que l'enveloppe de l'embase 20 est conformée pour recevoir le ventilateur 3 (Fig. 6A).

Ensuite, il s'opère un centrage du ventilateur 3 sur la station d'accueil 2 grâce au connecteur mâle 21 qui fait également office de système de centrage. Celui-ci peut comprendre à cette fin, une ou des géométries de centrage de type pion, cylindre, croisillon ou analogue, par exemple de 2 à 5 mm de diamètre, venant se loger dans l'empreinte du connecteur femelle 22 équipant le fond 9 du ventilateur 3 (Fig. 6B). On assiste aussi à un début de mise en contact des crochets 7 faisant alors translater le bouton-poussoir 26.

Enfin, une insertion totale du connecteur mâle 21 dans le connecteur femelle 22 assure une fin de centrage et l'établissement des contacts électriques (alimentations, signaux de pilotage...) et la connexion mécanique détaillée ci-avant, immobilise le ventilateur 3 sur l'embase 20 (Fig. 6C).

L'alimentation électrique de l'embase 20 de la station d'accueil 2, en particulier de la carte électronique 24 qui est aménagée dans l'embase 20, permet donc une ensuite une alimentation électrique du ventilateur 3, ainsi que l'alimentation et la recharge électrique de la ou des batteries, via une fonction chargeur de la carte électronique 24.

La Figure 9 montre également qu'est prévu un détrompage mécanique qui empêche de raccorder deux alimentations secteurs en même temps. En effet, lorsque le ventilateur 3 est positionné dans l'embase 20 de la station d'accueil 2, comme illustré en Fig. 9, une prise mâle de raccordement électrique 5 ne peut être enfichée que dans l'embase 20 de la station d'accueil 2 et une autre prise ne peut être enfichée en même temps, dans la prise femelle 39 du ventilateur 3 car la prise femelle 39 du ventilateur 3 est alors masquée par la protubérance 29 de l'embase 20 qui porte le bouton-poussoir 26.

L'embase 20 de la station d'accueil 2 peut comprendre aussi des pièces de parement et d'enveloppe, en matière plastique, et être conformée pour épouser le profil et/ou les formes extérieures du ventilateur 10 au niveau du premier emplacement 4a. En particulier, l'enveloppe de l'embase 20 comprend la protubérance 29 logeant le bouton-poussoir 26.

Par ailleurs, comme illustré en Figure 4, est aussi prévu sur la station d'accueil 2, une interface homme/machine ou IHM 12 comprenant au moins des voyants ou LEDs 'indicateurs' de bonne connexion de l'alimentation externe 12a, c'est-à-dire au secteur, d'éventuels défauts 12b, et d'états de charge de la (des) batterie 12c, par exemple trois barres de niveau de charge représentatives d'une batterie faible, en cours de rechargement ou chargée, et éventuellement un (des) bouton 12d permettant de commander la mise en marche ou l'arrêt du système, ou encore de vérifier le niveau de charge de la batterie. L'interface homme/machine peut comprendre aussi un écran de visualisation d'informations.

De préférence, la communication entre le ventilateur 3 et la (les) batterie d'alimentation électrique est assurée par la carte électronique 24 (cf. Fig. 5), laquelle carte 24 sert notamment de connexion de l'alimentation électrique externe, de sélecteur de source externe ou interne d'alimentation électrique du ventilateur, de chargeur de batteries capable de gérer différentes puissances (modes) de charge lorsque le ventilateur n'est pas en fonctionnement par exemple, à opérer une surveillance de l'état de l'alimentation externe et/ou de la ou des batteries ou une sélection d'une batterie pour la charge/décharge afin de maximiser l'autonomie de l'ensemble. La commutation entre les batteries connectées peut se faire pendant le fonctionnement de l'appareil, à la gestion de l'IHM 12, à la communication avec le ventilateur via un bus numérique, permettant l'échange d'information sur l'état de la station d'accueil, tel que l'alimentation, état des batteries..., tel que niveau de charge, batterie non reconnue, batterie à remplacer..., à la communication avec le ventilateur via un bus numérique, permettant l'échange d'information sur l'état du ventilateur, tel que la vitesse de la turbine, l'état de l'humidificateur, état des accessoires...

Par ailleurs, la carte électronique 24 peut aussi gérer une ou des batteries dites « intelligentes », c'est-à-dire connaissant son niveau de charge, son état... et pouvant communiquer ces informations via un bus numérique par exemple, ou une ou des batteries classiques. Dans le cas de batteries classiques, la carte doit estimer le niveau de charge des batteries, ainsi que les courants/tensions requis lors de la charge.

Le rechargement de la (ou des) batterie est préférentiellement autonome, c'est-à-dire que si la station contenant la (ou les) batterie est déconnectée du ventilateur, celle-ci se recharge automatiquement si elle est reliée électriquement au secteur.

On peut prévoir également des fonctions annexes au niveau de la station d'accueil 2 ou du ventilateur 3, comme l'ajout de ports de communication supplémentaires, tel un ou des ports USB, d'un système d'alarme complémentaire au ventilateur 3, un système de télécommunication ou de mise en réseau, ou encore la possibilité d'une connexion d'adjonction d'O₂ pour le ventilateur 3...

La station d'accueil 2 et le ventilateur respiratoire 3 et éventuellement l'humidificateur 4 de gaz, d'un ensemble de ventilation 1 selon l'invention est utilisable pour traiter des maladies respiratoires chez des patients humains, qu'ils soient adultes ou enfants, y compris les nouveau-nés.

## Revendications

1. Ensemble de ventilation (1) de patient comprenant une station d'accueil (2) et un ventilateur médical (3), dans lequel la station d'accueil (2) comprend des moyens d'accrochage (7, 13), et le ventilateur (3) comprend des moyens d'arrimage (8, 9) coopérant avec les moyens d'accrochage (7, 13) de la station d'accueil (2), lorsque le ventilateur (3) est positionné sur ladite station d'accueil (2), de manière à solidariser le ventilateur (3) à la station d'accueil (2), dans lequel :
- les moyens d'arrimage (8, 9) du ventilateur (3) comprennent au moins une ouverture (8) aménagée dans une paroi de fond (9) dudit ventilateur (3), et
- les moyens d'accrochage (7, 13) de la station d'accueil (2) comprennent au moins un élément à crochet (7), ledit au moins un élément à crochet (7) venant s'insérer dans ladite au moins une ouverture (8) du ventilateur (3) et prendre appui sur au moins une surface interne (9a) du ventilateur (3) pour maintenir ledit ventilateur (3) solidaire de la station d'accueil (2), **caractérisé en ce que** :
- le ou les éléments à crochet (7) sont mobiles en translation entre au moins :
i) une position de couplage dans laquelle le ou les éléments à crochet (7) prennent appui sur au moins une surface interne (9a) du ventilateur (3) pour maintenir ledit ventilateur (3) solidaire de la station d'accueil (2), et
ii) une position de découplage dans laquelle le ou les éléments à crochet (7) ne sont pas en appui sur une surface interne (9a) du ventilateur (3) de sorte de pouvoir désolidariser le ventilateur (3) de la station d'accueil (2), et
- les moyens d'accrochage (7) de la station d'accueil (2) comprennent en outre des moyens de rappel élastique (13) coopérant avec le ou les éléments à crochet (7) de manière à le ou les rappeler élastiquement en position de couplage.

2. Ensemble selon la revendication précédente, **caractérisé en ce que** les moyens d'arrimage (8, 9) du ventilateur (3) comprennent une pluralité d'ouvertures (8) aménagées dans la paroi de fond (9) dudit ventilateur (3), et les moyens d'accrochage (7, 13) de la station d'accueil (2) comprennent une pluralité d'éléments à crochet (7).

3. Ensemble selon l'une des revendications précédentes, **caractérisé en ce que** la ou les ouvertures (8) aménagées dans la paroi de fond (9) du ventilateur (3) sont des fentes.

4. Ensemble selon l'une des revendications précédentes, **caractérisé en ce que** les moyens de rappel élastique (13) comprennent au moins un ressort.

5. Ensemble selon l'une des revendications précédentes, **caractérisé en ce que** la station d'accueil (2) comprend en outre des moyens de libération (6), actionnables manuellement, coopérant avec les éléments à crochet (7) de manière à les déplacer vers la position de découplage, lorsqu'ils sont actionnés manuellement par un utilisateur.

6. Ensemble selon l'une des revendications précédentes, **caractérisé en ce que** les moyens de libération (6) comprennent un bouton-poussoir (26).

7. Ensemble selon l'une des revendications précédentes, **caractérisé en ce que** ledit au moins un élément à crochet (7) comprend une tête (14) avec une surface externe pentue (15).

8. Ensemble selon l'une des revendications précédentes, **caractérisé en ce que** la tête (14) à surface externe pentue ou arrondie (15) dudit au moins un élément à crochet (7) vient coopérer avec un rebord (9b) de ladite au moins une ouverture (8) aménagée dans la paroi de fond (9) du ventilateur (3), lorsque le ventilateur (3) est positionné par un utilisateur sur la station d'accueil (2), de manière à repousser mécaniquement ledit au moins élément à crochet (7) en direction de la position de découplage.

9. Ensemble selon l'une des revendications précédentes, **caractérisé en ce que** le nombre d'ouvertures (8) aménagées dans la paroi de fond (9) du ventilateur (3) est compris entre 2 et 10, de préférence de 3 à 6.

10. Ensemble selon l'une des revendications précédentes, **caractérisé en ce que** le nombre d'éléments à crochet (7) de la station d'accueil (2) est compris entre 2 et 10, de préférence de 3 à 6.

11. Ensemble selon l'une des revendications précédentes, **caractérisé en ce que** le nombre d'ouvertures (8) aménagées dans la paroi de fond (9) du ventilateur (3) est égal à 4 et le nombre d'éléments à crochet (7) de la station d'accueil (2) est égal à 4.

12. Ensemble selon l'une des revendications précédentes, **caractérisé en ce que** la station d'accueil (2) est conçue pour être positionnée horizontalement sur un support, en particulier un meuble.

13. Ensemble selon l'une des revendications précédentes, **caractérisé en ce que** le ou les éléments à crochet (7) sont mobiles en translation horizontale.

14. Ensemble selon l'une des revendications précédentes, **caractérisé en ce que** les moyens de libération (6) comprennent un bouton-poussoir (26) mobile en translation horizontale.

15. Ensemble selon l'une des revendications précédentes, **caractérisé en ce qu'**il comprend en outre un humidificateur de gaz (4) en communication fluidique avec le ventilateur (3).

## Patentansprüche

1. Patienten-Beatmungseinheit (1), welche eine Aufnahmevorrichtung (2) und ein medizinisches Beatmungsgerät (3) umfasst, wobei die Aufnahmevorrichtung (2) Einhakmittel (7, 13) umfasst und das Beatmungsgerät (3) Sicherungsmittel (8, 9) umfasst, die mit den Einhakmitteln (7, 13) der Aufnahmevorrichtung (2) zusammenwirken, wenn das Beatmungsgerät (3) auf der Aufnahmevorrichtung (2) positioniert wird, um das Beatmungsgerät (3) fest mit der Aufnahmevorrichtung (2) zu verbinden, wobei:
- die Sicherungsmittel (8, 9) des Beatmungsgerätes (3) wenigstens eine Öffnung (8) umfassen, die in einer Bodenwand (9) des Beatmungsgerätes (3) angeordnet ist, und
- die Einhakmittel (7, 13) der Aufnahmevorrichtung (2) wenigstens ein Hakenelement (7) umfassen, wobei das wenigstens eine Hakenelement (7) in die wenigstens eine Öffnung (8) des Beatmungsgerätes (3) eingesetzt wird und an wenigstens einer Innenfläche (9a) des Beatmungsgerätes (3) zur Anlage kommt, um das Beatmungsgerät (3) fest mit der Aufnahmevorrichtung (2) verbunden zu halten, **dadurch gekennzeichnet, dass**:
- das oder die Hakenelemente (7) translatorisch bewegbar sind zwischen wenigstens:
i) einer Kopplungsposition, in welcher das oder die Hakenelemente (7) an wenigstens einer Innenfläche (9a) des Beatmungsgerätes (3) anliegen, um das Beatmungsgerät (3) fest mit der Aufnahmevorrichtung (2) verbunden zu halten, und
ii) einer Entkopplungsposition, in welcher das oder die Hakenelemente (7) nicht an einer Innenfläche (9a) des Beatmungsgerätes (3) anliegen, so dass das Beatmungsgerät (3) von der Aufnahmevorrichtung (2) gelöst werden kann, und
- die Einhakmittel (7) der Aufnahmevorrichtung (2) außerdem elastische Rückstellmittel (13) umfassen, die mit dem oder den Hakenelementen (7) so zusammenwirken, dass sie dieses oder diese elastisch in die Kopplungsposition zurückstellen.

2. Einheit nach dem vorhergehenden Anspruch, **dadurch gekennzeichnet, dass** die Sicherungsmittel (8, 9) des Beatmungsgerätes (3) mehrere Öffnungen (8) umfassen, die in der Bodenwand (9) des Beatmungsgerätes (3) angeordnet sind, und die Einhakmittel (7, 13) der Aufnahmevorrichtung (2) mehrere Hakenelemente (7) umfassen.

3. Einheit nach einem der vorhergehenden Ansprüche, **dadurch gekennzeichnet, dass** die Öffnung oder die Öffnungen (8), die in der Bodenwand (9) des Beatmungsgerätes (3) angeordnet sind, Schlitze sind.

4. Einheit nach einem der vorhergehenden Ansprüche, **dadurch gekennzeichnet, dass** die elastischen Rückstellmittel (13) wenigstens eine Feder umfassen.

5. Einheit nach einem der vorhergehenden Ansprüche, **dadurch gekennzeichnet, dass** die Aufnahmevorrichtung (2) außerdem manuell betätigbare Freigabemittel (6) umfasst, die mit den Hakenelementen (7) so zusammenwirken, dass sie diese in die Entkopplungsposition verlagern, wenn sie von einem Benutzer manuell betätigt werden.

6. Einheit nach einem der vorhergehenden Ansprüche, **dadurch gekennzeichnet, dass** die Freigabemittel (6) eine Drucktaste (26) umfassen.

7. Einheit nach einem der vorhergehenden Ansprüche, **dadurch gekennzeichnet, dass** das wenigstens eine Hakenelement (7) einen Kopf (14) mit einer schrägen Außenfläche (15) umfasst.

8. Einheit nach einem der vorhergehenden Ansprüche, **dadurch gekennzeichnet, dass** der Kopf (14) mit schräger oder abgerundeter Außenfläche (15) des wenigstens einen Hakenelements (7) mit einem Rand (9b) der wenigstens einen Öffnung (8), die in der Bodenwand (9) des Beatmungsgerätes (3) angeordnet ist, zusammenwirkt, wenn das Beatmungsgerät (3) von einem Benutzer auf der Aufnahmevorrichtung (2) positioniert wird, derart, dass das wenigstens eine Hakenelement (7) mechanisch in Richtung der Entkopplungsposition zurückgeschoben wird.

9. Einheit nach einem der vorhergehenden Ansprüche, **dadurch gekennzeichnet, dass** die Anzahl der Öffnungen (8), die in der Bodenwand (9) des Beatmungsgerätes (3) angeordnet sind, zwischen 2 und 10 liegt, vorzugsweise zwischen 3 und 6.

10. Einheit nach einem der vorhergehenden Ansprüche, **dadurch gekennzeichnet, dass** die Anzahl der Hakenelemente (7) der Aufnahmevorrichtung (2) zwischen 2 und 10 liegt, vorzugsweise zwischen 3 und 6.

11. Einheit nach einem der vorhergehenden Ansprüche, **dadurch gekennzeichnet, dass** die Anzahl der Öffnungen (8), die in der Bodenwand (9) des Beatmungsgerätes (3) angeordnet sind, gleich 4 ist und die Anzahl der Hakenelemente (7) der Aufnahmevorrichtung (2) gleich 4 ist.

12. Einheit nach einem der vorhergehenden Ansprüche, **dadurch gekennzeichnet, dass** die Aufnahmevorrichtung (2) dafür ausgelegt ist, horizontal auf einem Träger, insbesondere einem Möbelstück, positioniert zu werden.

13. Einheit nach einem der vorhergehenden Ansprüche, **dadurch gekennzeichnet, dass** die Hakenelemente (7) horizontal translatorisch bewegbar sind.

14. Einheit nach einem der vorhergehenden Ansprüche, **dadurch gekennzeichnet, dass** die Freigabemittel (6) eine Drucktaste (26) umfassen, die horizontal translatorisch bewegbar ist.

15. Einheit nach einem der vorhergehenden Ansprüche, **dadurch gekennzeichnet, dass** sie außerdem einen Gasbefeuchter (4) umfasst, der mit dem Beatmungsgerät (3) in Fluidverbindung steht.

## Claims

1. Patient ventilation assembly (1) comprising a docking station (2) and a medical ventilator (3), in which the docking station (2) comprises hooking means (7, 13), and the ventilator (3) comprises securing means (8, 9) interacting with the hooking means (7, 13) of the docking station (2) when the ventilator (3) is positioned on said docking station (2), so as to rigidly connect the ventilator (3) to the docking station (2), in which:
- the securing means (8, 9) of the ventilator (3) comprise at least one opening (8) formed in a bottom wall (9) of said ventilator (3), and
- the hooking means (7, 13) of the docking station (2) comprise at least one hook element (7), said at least one hook element (7) being inserted into said at least one opening (8) of the ventilator (3) and bearing on at least one inner surface (9a) of the ventilator (3) in order to maintain said ventilator (3) rigidly connected to the docking station (2),
**characterized in that**:
- the one or more hook elements (7) are movable in translation between at least:
i) a coupling position, in which the one or more hook elements (7) bear on at least one inner surface (9a) of the ventilator (3) in order to maintain said ventilator (3) rigidly connected to the docking station (2), and
ii) a decoupling position, in which the one or more hook elements (7) do not bear on an inner surface (9a) of the ventilator (3), so as to be able to detach the ventilator (3) from the docking station (2), and
- the hooking means (7) of the docking station (2) further comprise elastic return means (13) that interact with the one or more hook elements (7) so as to return them elastically to the coupling position.

2. Assembly according to the preceding claim, **characterized in that** the securing means (8, 9) of the ventilator (3) comprise a plurality of openings (8) formed in the bottom wall (9) of said ventilator (3), and the hooking means (7, 13) of the docking station (2) comprise a plurality of hook elements (7).

3. Assembly according to either of the preceding claims, **characterized in that** the one or more openings (8) formed in the bottom wall (9) of the ventilator (3) are slots.

4. Assembly according to one of the preceding claims, **characterized in that** the elastic return means (13) comprise at least one spring.

5. Assembly according to one of the preceding claims, **characterized in that** the docking station (2) further comprises release means (6), which are actuatable manually, and which interact with the hook elements (7) so as to move the latter to the decoupling position, when they are actuated manually by a user.

6. Assembly according to one of the preceding claims, **characterized in that** the release means (6) comprise a push button (26).

7. Assembly according to one of the preceding claims, **characterized in that** said at least one hook element (7) comprises a head (14) with a sloping outer surface (15).

8. Assembly according to one of the preceding claims, **characterized in that** the head (14), with sloping or rounded outer surface (15), of said at least one hook element (7) interacts with an edge (9b) of said at least one opening (8) formed in the bottom wall (9) of the ventilator (3), when the ventilator (3) is positioned by a user on the docking station (2), so as to mechanically push said at least one hook element (7) in the direction of the decoupling position.

9. Assembly according to one of the preceding claims, **characterized in that** the number of openings (8) formed in the bottom wall (9) of the ventilator (3) is between 2 and 10, preferably from 3 to 6.

10. Assembly according to one of the preceding claims, **characterized in that** the number of hook elements (7) of the docking station (2) is between 2 and 10, preferably from 3 to 6.

11. Assembly according to one of the preceding claims, **characterized in that** the number of openings (8) formed in the bottom wall (9) of the ventilator (3) is equal to 4 and the number of hook elements (7) of the docking station (2) is equal to 4.

12. Assembly according to one of the preceding claims, **characterized in that** the docking station (2) is designed to be positioned horizontally on a support, in particular an item of furniture.

13. Assembly according to one of the preceding claims, **characterized in that** the one or more hook elements (7) are movable in horizontal translation.

14. Assembly according to one of the preceding claims, **characterized in that** the release means (6) comprise a push button (26) movable in horizontal translation.

15. Assembly according to one of the preceding claims, **characterized in that** it further comprises a gas humidifier (4) in fluidic communication with the ventilator (3).
